# EUROPEAN PATENT APPLICATION

(11) **EP 0 794 175 A2**
(43) Date of publication of application: **10.09.1997**
(21) Application number: 96308380.3
(22) Date of filing: 19.11.1996
(51) Int. Cl.: C07C 401/00, A61K 31/59

(54) **Process for producing vitamin D3 derivatives**

(30) Priority: 20.11.1995 JP 325128/95
(71) Applicant: KUREHA KAGAKU KOGYO KABUSHIKI KAISHA, Chuo-ku Tokyo 103 (JP)
(72) Inventor: Maruoka, Hiroshi, Setagaya-ku, Tokyo (JP); Tatsuta, Kuniaki, Tokyo (JP); Yanaka, Mikiro, Matsudo-shi, Chiba-ken (JP)
(74) Representative: Woods, Geoffrey Corlett

(57) **Abstract**

A process for producing a 23-hydroxy-24-oxovitamin D₃ derivative which comprises oxidizing a 24-hydroxyvitamin D₃, derivative to form a 24-oxovitamin D₃ derivative, silyl-enol-ethyerifying the 24-oxovitamin D₃ derivative to form an etherified compound, oxidizing the etherified compound, and subjecting the resulting compound to a hydroxyl-protecting group elimination reaction.

## Description

The present invention relates to a process for producing vitamin D₃ derivatives, and more particularly, to a process for producing 23-hydroxy-24-oxovitamin D₃ derivatives.

23-hydroxy-24-oxovitamin D₃ derivatives are the important compounds for use as a standard reagent in the study of metabolism of vitamin D₃, but scanty supply of these derivatives has hindered the studies on their pharmacological effects and properties such as toxicity, so that the development of the efficient preparation process of these derivatives has been desired.

It is known that 23,25-dihydroxy-24-oxocholecalciferol which is a metabolite of 24R,25-dihydroxycholecalciferol, can be obtained from a complicate multistage chemical synthetic process using 25-hydroxy-24-oxo-7-dehydrocholesterol as starting material and comprising the steps of protecting the diene units in its chemical structure with 4-phenyl-1,2,4-triazoline-3,5-dione, oxidizing the side chains of the provitamin, eliminating the protective groups of diene, and causing ring-opening of the compound by photoreaction (S. Yamada et al: Biochemistry, Vol. 28, No. 11, pp. 4551-4556 (1989)).

A more simple method using an enzyme system is disclosed in Japanese Patent Application Laid-Open (KOKAI) No. 6-205685. According to this method, the electron transmission system is reconstructed by using an enzyme produced from a mitochondria P450_{C24}-producing E. coli strain and bovine adrenal ADX and ADR necessary for manifestation of activity of said enzyme, and the substrate 24,25-dihydroxycholecalciferol is converted into 23,25-dihydroxy-24-oxocholecalciferol by using said reconstructed system.

However, since the above-mentioned chemical synthetic process is a multistage reaction system, overall yield from the starting material is as low as about 7%. Further, since the starting material itself is synthesized from hyodeoxycholic acid through a multistage reaction scheme, total yield of the product is too low. On the other hand, in the method using an enzyme system, although the conversion percentage is as relatively high as 31%, a complicate enzyme reconstruction system is required and also there is a problem such as inadaptability to mass synthesis since the conversion reaction is carried out in a dilute substrate solution.

As a result of the present inventors' earnest studies for solving the above problems, it has been found that by using 24-hydroxyvitamin D₃ derivatives, which are easily available in large quantities, as starting material with a simple process comprising carrying out a hydroxyl group oxidation reaction, a silylenol etherification reaction, an oxidation reaction and a protective group elimination reaction, it is possible to mass-produce 23-hydroxy-24-oxovitamin D₃ derivatives with ease in a high yield. The present invention has been attained on the basis of this finding.

The object of the present invention is to provide a process for producing 23-hydroxy-24-oxovitamin D₃ derivatives in large quantities with ease in a high yield by using 24-hydroxyvitamin D₃ derivatives which are commercially available in large quantities, as starting material.

The another object of the present invention is to provide a process for producing 24-oxovitamin D₃ derivatives by using 24-hydroxyvitamin D₃ derivatives.

The still another object of the present invention is to provide a process for producing 23-hydroxy-24-oxovitamin D₃ derivatives by using 24-oxovitamin D₃ derivatives.

The further another object of the present invention is to provide a process for producing 23-hydroxyvitamin D₃ derivatives whose hydroxyl group alone on the ring is selectively protected by using silyl compounds.

To accomplish the aims, in a first aspect of the present invention, there is provided a process for producing vitamin D₃ derivatives represented by the following formula (I), which comprises oxidizing a compound represented by the following formula (V) to form a compound represented by the following formula (IV), silyl-enol-etherifying the obtained compound with an enolizing agent and a silylating agent, oxidizing the etherified compound with a peracid, and subjecting the resulting compound to a hydroxyl-protecting group elimination reaction: 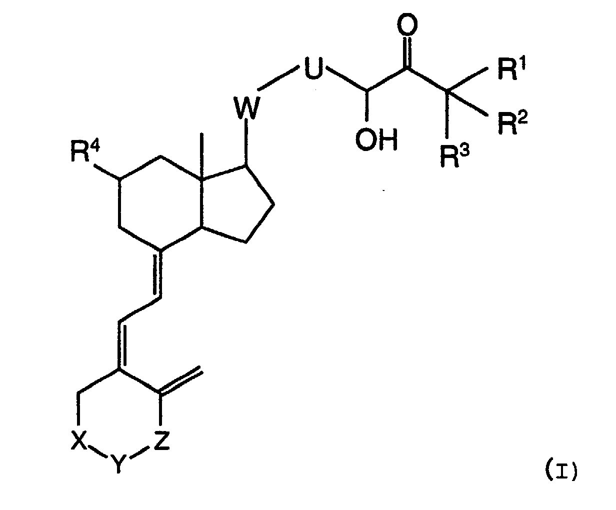 wherein R¹ represents an alkyl group, a hydroxyl group, a halogen atom, a hydroxyalkyl group or a halogenated alkyl group; R² and R³ represent independently an alkyl group, -OR⁶ (R⁶ being a hydroxyl-protecting group), a halogen atom, a hydroxyalkyl group or a halogenated alkyl group, or R² and R³ combine to form -O(CH₂)ₙ-, -O(CH₂)ₙO- or -(CH₂)ₙ₊₁- (n being an integer of not less than 1, preferably 1 to 10, more preferably 1 to 5); R⁴ represents a hydrogen atom, an alkyl group, a hydroxyl group, a halogen atom, a hydroxyalkyl group, a halogenated alkyl group or an aryl group; U, W, X, Y and Z represent independently -(S=O)-, -O-, -(CR⁷R⁸)- or -(CR⁷R⁸-CR⁹R¹⁰)- (wherein R⁷, R⁸, R⁹ and R¹⁰ represent independently a hydrogen atom, an alkyl group, a hydroxyl group, a halogen atom, a hydroxyalkyl group or a halogenated alkyl group; in case where a hydroxyl group is contained in any of U, W, X, Y and Z, the hydroxyl group is not protected; in U, W, X, Y and Z, -O- or -(S=O)- does not exist in succession); 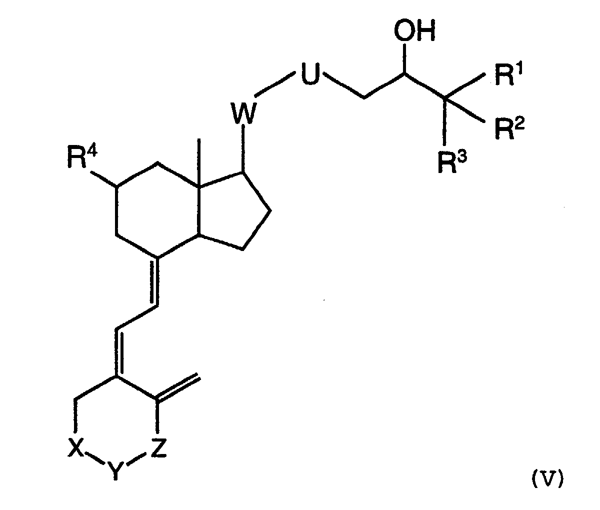 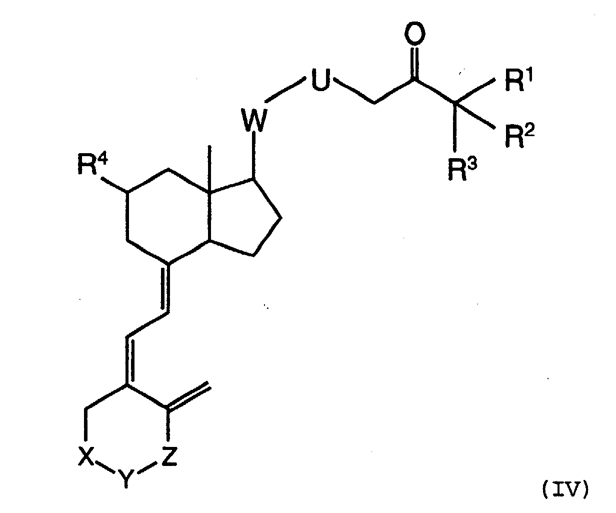 wherein R¹ represents an alkyl group, -OR⁵ (R⁵ being a hydrogen atom or a hydroxyl-protecting group), a halogen atom, a hydroxyalkyl group or a halogenated alkyl group; R² and R³ represent independently an alkyl group, -OR⁶ (R⁶ being a hydroxyl-protecting group), a halogen atom, a hydroxyalkyl group or a halogenated alkyl group, or R² and R³ combine to form -O(CH₂)ₙ-, -O(CH₂)ₙO- or -(CH₂)ₙ₊₁- (n being an integer of not less than 1, preferably 1 to 10, more preferably 1 to 5); R⁴ represents a hydrogen atom, an alkyl group, -OR⁵, a halogen atom, a hydroxyalkyl group, a halogenated alkyl group or an aryl group; U, W, X, Y and Z represent independently -(S=O)-, -O-, -(CR⁷R⁸)- or -(CR⁷R⁸-CR⁹R¹⁰)- (wherein R⁷, R⁸, R⁹ and R¹⁰ represent independently a hydrogen atom, an alkyl group, -OR⁵, a halogen atom, a hydroxyalkyl group or a halogenated alkyl group; in case where a hydroxyl group is contained in any of U, W, X, Y and Z, this group is protected; also, in U, W, X, Y and Z, -O- or -(S=O)- does not exist in succession).

In a second aspect of the present invention, there is provided a process for producing vitamin D₃ derivatives represented by the formula (IV) which comprises oxidizing a compound represented by the formula (V): 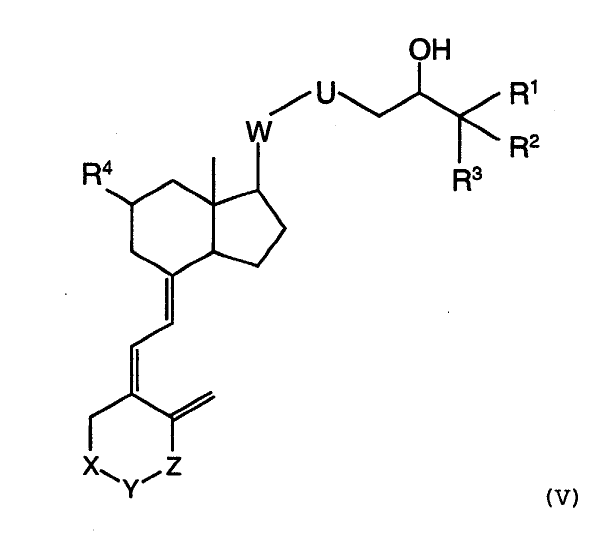 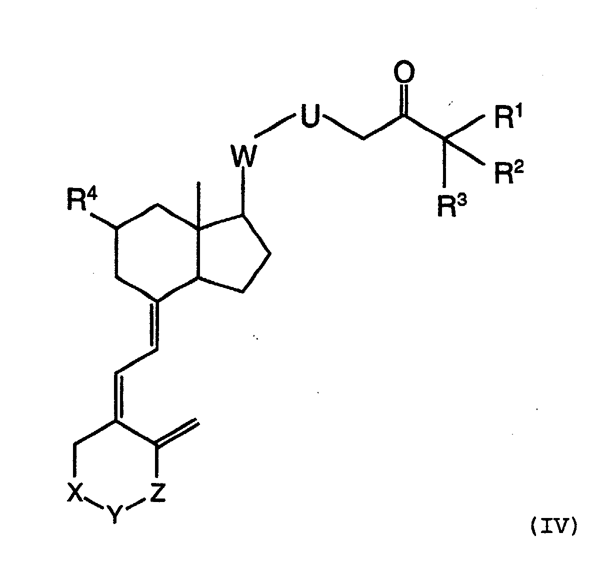 wherein R¹ represents an alkyl group, -OR⁵ (R⁵ being a hydrogen atom or a hydroxyl-protecting group), a halogen atom, a hydroxyalkyl group or a halogenated alkyl group; R² and R³ represent independently an alkyl group, -OR⁶ (R⁶ being a hydroxyl-protecting group), a halogen atom, a hydroxyalkyl group or a halogenated alkyl group, or R² and R3 combine to form -O(CH₂)ₙ-, -O(CH₂)ₙO- or -(CH₂)ₙ₊₁- (n being an integer of not less than 1, preferably 1 to 10, more preferably 1 to 5); R⁴ represents a hydrogen atom, an alkyl group, -OR⁵, a halogen atom, a hydroxyalkyl group, a halogenated alkyl group or an aryl group; U, W, X, Y and Z represent independently -(S=O)-, -O-, -(CR⁷R⁸)- or -(CR⁷R⁸-CR⁹R¹⁰)- (wherein R⁷, R⁸, R⁹ and R¹⁰ represent independently a hydrogen atom, an alkyl group, -OR⁵, a halogen atom, a hydroxyalkyl group or a halogenated alkyl group; in case where a hydroxyl group is contained in any of U, W, X, Y and Z, this group is protected; also, in U, W, X, Y and Z, -O- or -(S=O)- does not exist in succession).

In a third aspect of the present invention, there is provided a process for producing vitamin D3 derivatives represented by the formula (I) which comprises silyl-enol etherifying a compound represented by the above-shown formula (IV) with an enolizing agent and a silylating agent, oxidizing the etherified compound with a peracid, and subjecting the resulting compound to a hydroxyl-protecting group elimination reaction: 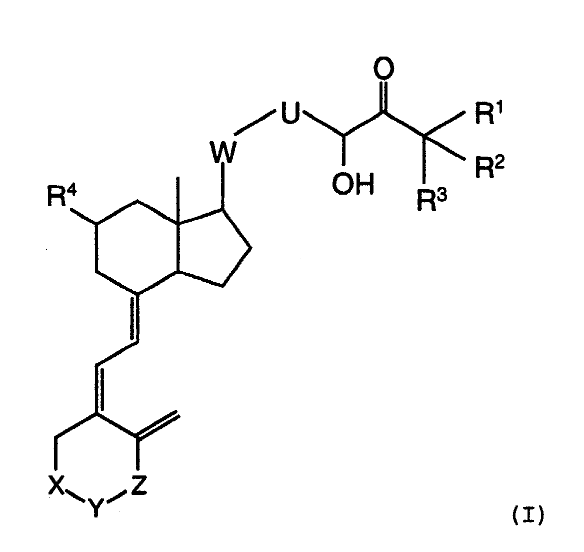 wherein R¹ represents an alkyl group, a hydroxyl group, a halogen atom, a hydroxyalkyl group or a halogenated alkyl group; R² and R³ represent independently an alkyl group, -OR⁶ (R⁶ being a hydroxyl-protecting group), a halogen atom, a hydroxyalkyl group or a halogenated alkyl group, or R² and R³ combine to form -O(CH₂)ₙ-, -O(CH₂)ₙO- or -(CH₂)ₙ₊₁- (n being an integer of not less than 1, preferably 1 to 10, more preferably 1 to 5); R⁴ represents a hydrogen atom, an alkyl group, a hydroxyl group, a halogen atom, a hydroxyalkyl group, a halogenated alkyl group or an aryl group; U, W, X, Y and Z represent independently -(S=O)-, -O-, -(CR⁷R⁸)- or -(CR⁷R⁸-CR⁹R¹⁰)- (wherein R⁷, R⁸, R⁹ and R¹⁰ represent independently a hydrogen atom, an alkyl group, a hydroxyl group, a halogen atom, a hydroxyalkyl group or a halogenated alkyl group; in case where a hydroxyl group is contained in any of U, W, X, Y and Z, the hydroxyl group is not protected; in U, W, X, Y and Z, -O- or -(S=O)- does not exist in succession).

In a fourth aspect of the present invention, there is provided a process for producing vitamin D3 derivatives represented by the formula (Vb) which comprises reacting a compound represented by the formula (Va) with a compound represented by the formula (VII):

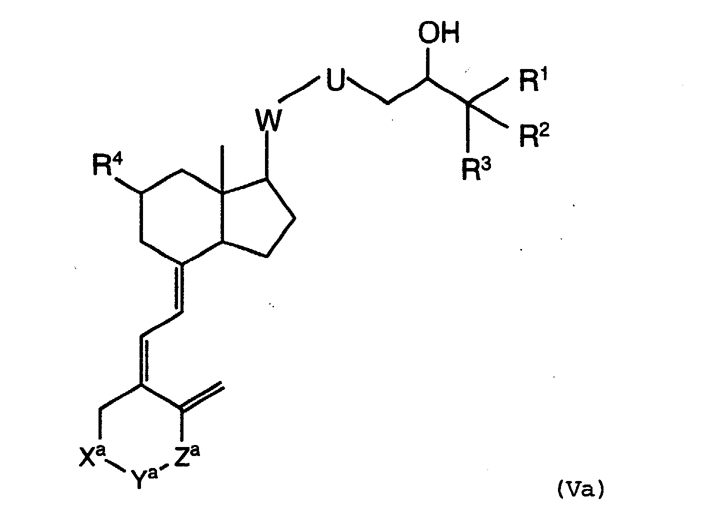

R¹¹R¹²R¹³Si-J (VII)

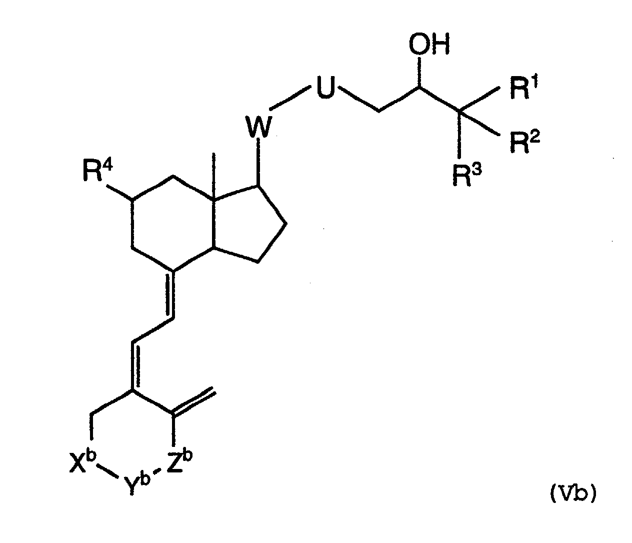

wherein, in the formulae (Va) and (Vb), R¹ represents an alkyl group, -OR⁵ (R⁵ being a hydrogen atom or a hydroxyl-protecting group), a halogen atom, a hydroxyalkyl group or a halogenated alkyl group; R² and R³ represent independently an alkyl group, -OR⁶ (R⁶ being a hydroxyl-protecting group), a halogen atom, a hydroxyalkyl group or a halogenated alkyl group, or R² and R3 combine to form -O(CH₂)ₙ-, -O(CH₂)ₙO- or -(CH₂)ₙ₊₁- (n being an integer of not less than 1, preferably 1 to 10, more preferably 1 to 5); R⁴ represents a hydrogen atom, an alkyl group, -OR⁵, a halogen atom, a hydroxyalkyl group, a halogenated alkyl group or an aryl group; U, W, X^{a}, Y^{a} and Z^{a}, X^{b}, Y^{b}, Z^{b} represent independently -(S=O)-, -O-, -(CR⁷R⁸)- or -(CR⁷R⁸-CR⁹R¹⁰)- (wherein R⁷, R⁸, R⁹ and R¹⁰ represent independently a hydrogen atom, an alkyl group, -OR⁵, a halogen atom, a hydroxyalkyl group or a halogenated alkyl group); at least one of X^{a}, Y^{a} and Z^{a} is -CH(OH)- and at least one of X^{b}, Y^{b} and Z^{b} is -CH(OSiR¹³R¹²R¹¹)- (wherein R¹¹, R¹² and R¹³ represent independently an alkyl group having 1 to 6 carbon atoms); in the formula (VII), J represents a halogen atom and -SiR¹³R¹²R¹¹ represents a hydroxyl-protecting group, and in U,W, X^{a}, Y^{a} and Z^{a} or in U, W, X^{b}, Y^{b} and Z^{b}, -O- or -(S=O)- does not exist in succession.

The present invention is described in more detail herein below.

In the compounds represented by the formulae (I), (IV), (V), (Va), (Vb) and (VII) according to the present invention, the alkyl group in R¹, R², R³, R⁴, R⁷, R⁸, R⁹ and R¹⁰ may be either linear or branched and has usually 1 to 6, preferably 1 to 4 carbon atoms. Examples of such alkyl group include methyl, ethyl, n-propyl, i-propyl, n-butyl, i-butyl, s-butyl, t-butyl, n-pentyl, i-pentyl, neopentyl, t-pentyl, 1-methylbutyl, 2-methylbutyl, 1,2-dimethylpropyl, 1-ethylpropyl, n-hexyl, i-hexyl and 2-ethylbutyl. Of these alkyl groups, methyl group or ethyl group is preferable and methyl group is more preferable. R² and R³ may combine to form -O(CH₂)ₙ-, -O(CH₂)ₙO- or -(CH₂)ₙ₊₁- wherein n is an integer of not less than 1, preferably 1 to 10, more preferably 1 to 5.

In the present invention, the hydroxyl-protecting group may be an ordinary protective group such as benzyl, acyl, silyl, etc., and a silyl group containing at least one alkyl group having 1 to 6 carbon atoms is preferred, and a silyl group containing three alkyl groups having 1 to 6 carbon atoms independently of each other is more preferred. The halogen atom may be, for instance, chlorine atom, bromine atom, fluorine atom or iodine atom.

In the compounds of the present invention, the hydroxyalkyl group is an alkyl group, preferably an alkyl group having 1 to 6 carbon atoms, having at least one hydroxyl group. Preferred examples of such hydroxyalkyl group include hydroxymethyl, 1-hydroxyethyl, 2-hydroxyethyl, 1-hydroxypropyl, 2-hydroxypropyl and 3-hydroxypropyl.

In the compound of the present invention, hydrogenated alkyl group is an alkyl group having 1 to 6 carbon atoms and at least one halogen atom. Preferred examples of such halogenated alkyl group is trifluoromethyl group, pentafluoroethyl group and 4,4,4-trifluorobutyl group.

The aryl group in the present invention may be a substituted aryl group having a substituent group such as alkyl, halogen, hydroxyl, etc., in an aromatic hydrocarbon. Preferred examples of such aryl group include phenyl, tolyl and naphthyl.

U in the side chain in the compounds (IV), (V), (Va) and (Vb) and in the vitamin D₃ derivatives (I) is preferably -(CR⁷R⁸)-. More preferably, both U and W are independently -(CR⁷R⁸)-.

The process according to the present invention is now explained. In the step of oxidizing a compound of the formula (V) to obtain a compound of the formula (IV), there is used an oxidizing agent which does not easily become acidic, in consideration of poor acid and heat resistance of the triene structure. Preferred examples of such oxidizing agent include sulfur trioxide-pyridine (SO₃ • Py), manganese dioxide and oxalyl dichloride-dimethyl sulfoxide ((COCl)₂ • DMSO; Swern oxidation). SO₃ · Py is especially preferred. In case of using SO₃ • Py as oxidizing agent, the reaction is preferably carried out in the presence of an amine compound such as triethylamine in an organic solvent such as DMSO at 0 to 25°C. When the reaction temperature is less than 0°C, the rate of reaction may be reduced. When the reaction temperature is more than 25°C, the yield may decrease.

In case where at least one of X, Y and Z in a ring of the compound of the formula (V) is -CH(OH)-, that is, in the case of the compound of the formula (Va), it needs to protect the hydroxyl group on this ring to an oxidizing agent. In this case, by using a silyl compound of the formula (VII), especially t-butyldimethylsilyl chloride (TBDMSCl), it is possible to selectively protect the hydroxyl group alone on the ring while leaving unprotected the hydroxyl group in the side chain which is the target of oxidation, thereby obtaining a compound of the formula (Vb). This reaction is preferably carried out in the presence of a base in an organic solvent such as dimethylformamide at 0 to 25°C. It is preferred to use an amine such as imidazole as the base. When the reaction temperature is less than 0°C, the rate of reaction may be reduced. When the reaction temperature is more than 25°C, the yield may decrease.

As the enolizing agent used in the step of silyl-enol etherifying the compound of the formula (IV) with an enolizing agent and a silylating agent, there can be used, for example, lithium diisopropylamide (LDA), sodium hydride, potassium hexamethyldisilazide and the like. As the silylating agent of the formula (VII), TBDMSCl, trimethylsilyl chloride (TMSCl) and the like can be used. LDA is most preferred as the enolizing agent, and TBDMSCl is most preferable as the silylating agent.

The above reaction is preferably carried out in an organic solvent such as tetrahydrofuran, hexamethylphosphoramide, etc., or a mixture thereof at a temperature in the range from -50°C to 25°C by adding an enolizing agent and a silylating agent. In this reaction, the hydroxyl groups which have existed to this stage are also silyl-etherified. When the reaction temperature is less than 50°C, the rate of reaction may be reduced. When the reaction temperature is more than 25°C, the yield may decrease.

As the peracid used in the step of oxidizing the silyl-enol ether, there can be used all types of peracid which are capable of epoxydizing the enol double bonds. Typical examples of such peracid are performic acid, peracetic acid, perbenzoic acid, m-chloroperbenzoic acid (mCPBA), perphthalic acid and the like. Of these peracids, perbenzoic acid and mCPBA are preferred, the latter being especially preferred. In this reaction, the silyl-enol ether once becomes an intermediary epoxide and then is made into an α-silyloxyketone, accompanied by rearrangement of the silyl groups.

The above reaction is preferably carried out in an organic solvent such as methylene chloride at 0 to 25°C. In this reaction, the peracid is preferably used in a molar ratio of 0.8 to 1.1 to the silyl-enol ether. When the ratio of the peroxide is more than 1.1, the double bonds other than those of the enol, that is, the triene double bonds may be also epoxidized and the yield of the objective compound may decrease. When the ratio of the peroxide is less than 0.8, the yield may decrease.

After oxidation with a peracid, the silyl group of the silyl ether, the silyl group introduced to the hydroxyl group present in the silyl-enol ether forming reaction and the hydroxyl-protecting group are eliminated to obtain a vitamin D₃ derivative of the formula (I). The hydroxyl-protecting group is in most cases a silyl group. This reaction is preferably carried out in the presence of a desilylating agent such as tetrabutylammonium fluoride (TBAF) in an organic solvent such as tetrahydrofuran at 0 to 25°C. When the reaction temperature is less than 0°C, the rate of reaction may be reduced. When the reaction temperature is more than 25°C, the yield may decrease.

The reaction product may be purified by the various methods such as extraction, chromatography, crystallization and reprecipitation. The 23R-form and the 23S-form may be separated by liquid chromatography or other suitable means. The chemical structure of the reaction product can be confirmed in various ways such as IR absorption spectrum, UV absorption spectrum, NMR spectrum, elementary analysis and mass spectrum.

According to the process of the present invention described above, various kinds of 23-hydroxy-24-oxovitamin D₃ derivatives can be obtained. An example of synthetic scheme for obtaining 23,25-dihydroxy-24-oxocholecalciferol (I-1) from 24,25-dihydroxycholecalciferol (Va-1) is shown below.

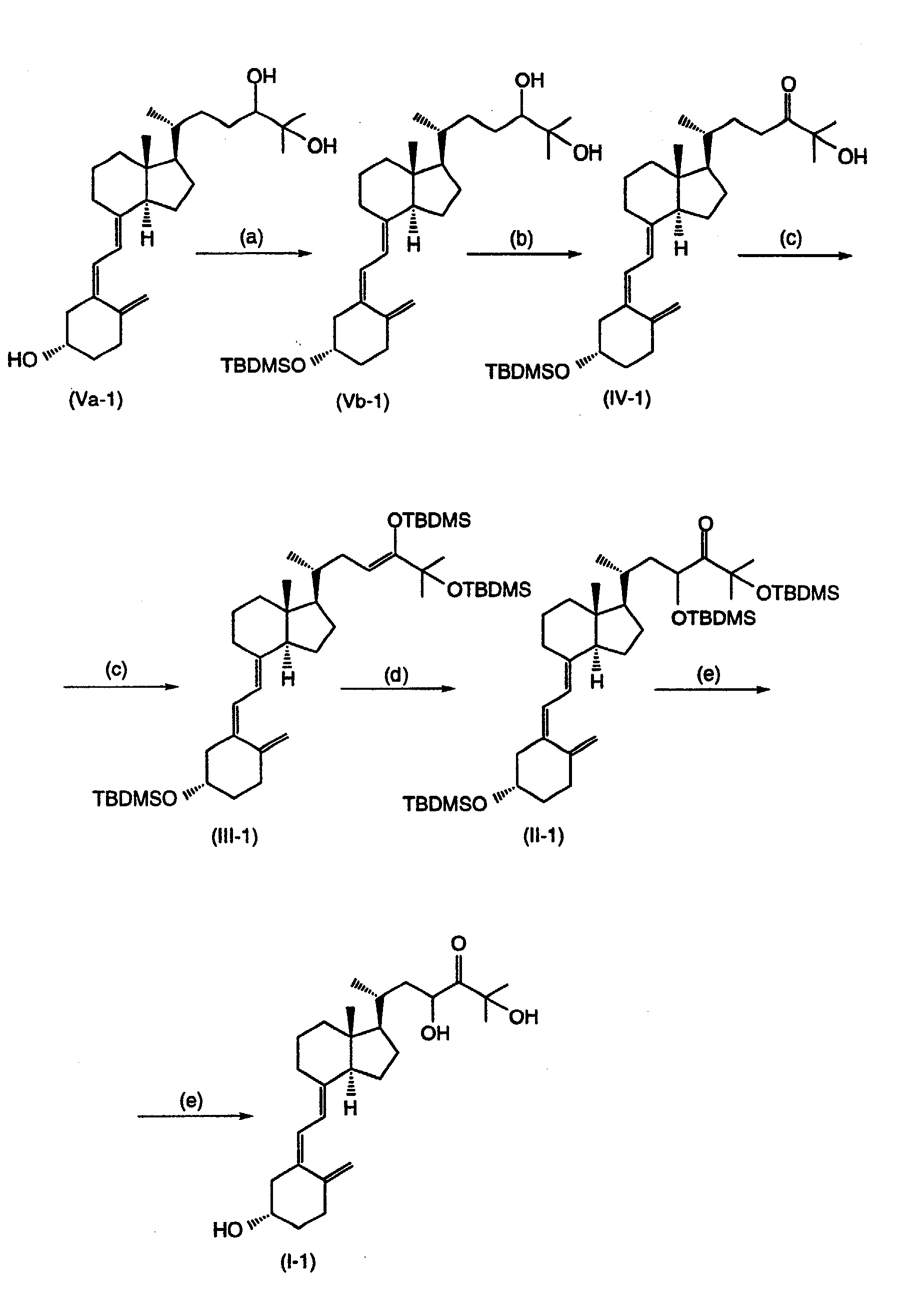

The above synthetic process is explained stepwise below.

### Step (a):

This step comprises introducing a protective group to the hydroxyl group at the 3-position of 24,25-dihydroxycholecalciferol (Va-1). The hydroxyl group at the 25-position is tertiary and the hydroxyl groups at the 3-position and the 24-position are secondary, so that both of the hydroxyl groups at the 3-position and the 24-position are acetylated under the normal reaction conditions used for introducing an acetyl group as a protective group.

On the other hand, in case where the protective group is a t-butyldimethylsilyl (TBDMS) group, the hydroxyl group at the 3-position is selectively protected in the form of silyl ether while the hydroxyl group at the 24-position, which is the target hydroxyl group for oxidation of the next stage, is not protected, and 3-TBDMS-24,25-dihydroxycholecalciferol (Vb-1) is obtained in the manner described in Example 1 shown later.

The 24,25-dihydroxycholecalciferol used as starting material is a known compound which is described in, for instance, Pharmacia, Vol. 10, No. 5, pp. 319-320 (1974), and there exist its isomers 24R,25-dihydroxycholecalciferol and 24S,25-dihydroxycholecalciferol. In the present invention, the 24R-form, the 24S-form, mixtures thereof at various proportions and their racemates can be used.

### Step (b):

In this step, the hydroxyl group at the 24-position is oxidized into 24-oxo. As described in Example 2 shown below, 3-TBDMS-24-oxo-25-hydroxycholecalciferol (IV-1) can be obtained in a high yield by using SO₃ · Py without protecting the triene structure which is highly reactive. Such a fact was found out for the first time by the present inventors.

### Step (c):

This is the step for obtaining silyl-enol ether (III-1) from the compound (IV-1) by using an enolizing agent and a silylating agent. In this reaction, 24-oxo undergoes enolization and silylation, and at the same time the hydroxyl group at the 25-position is also silylated to form a silyl ether. (See Example 3).

### Step (d):

This step comprises oxidizing silyl-enol ether (III-1) with a peracid to form a tris(t-butyldimethylsilyl)ether (II-1) of 23,25-dihydroxy-24-oxocholecalciferol. In this step, usually a mixture of 23R-form and 23S-form is obtained. (See Example 4).

### Step (e):

This step comprises eliminating the silyl groups from the tris(t-butyldimethylsilyl)ether (II-1) to give the objective 23,25-dihydroxy-24-oxocholecalciferol (I-1) . (See Example 4).

23-oxo-24-hydroxycholecalciferol can be obtained by eliminating TBDMS group at the 3-position of the compound (IV-1) obtained in the step (b). This reaction is carried out in the same manner as in the step (e).

It is quite a surprising and significant achievement which breaks through the conventional concept, to attain a high yield in the above process by carrying out the reactions without protecting the triene structure which is susceptible to acids and heat, and is highly reactive to various reagents. This surprising effect could be attained only through the embodiment of the present invention.

According to the present invention, as described above, there is provided a process for producing 23-hydroxy-24-oxovitamin D₃ derivatives, which process is simple and capable of commercial production of said derivatives in a high yield, by using as starting material 24-hydroxyvitamin D₃ derivatives which are obtainable in large quantities.

### EXAMPLES

The present invention is further illustrated by the following examples. These examples, however, are merely intended to be illustrative and not to be construed as limiting the scope of the invention.

### Example 1

### <Synthesis of (24R)-3-O-t-butyldimethylsilyl-24,25-dihydroxycholecalciferol>

To a solution of 24R,25-dihydroxycholecalciferol (500 mg) in dimethylformamide (DMF) (5 ml), imidazole (246 mg) and t-butyldimethylsilyl chloride (TBDMSCl) (273 mg) were added and stirred at room temperature for 3 hours. Water was added to the resulting reaction solution and the solution was extracted thrice with chloroform (30 ml).

The recovered organic layer was concentrated under reduced pressure and the residue was purified by silica gel column chromatography (silica gel: 20 g; solvent: n-hexane/ ethyl acetate (4/1 v/v)) to give the caption compound (637 mg; yield: 100%) as a colorless syrup. The parent peak mass of the mass spectrum was 530, and NMR determinations gave the following results.
¹H-NMR (500 MHz, CDCl₃) δ: 6.16 and 6.01 (2H, 2d, H-6 and 7, J = 11.5 Hz), 5.01 and 4.78 (2H, 2s, H-19), 3.82 (1H, m, H-3), 3.33 (1H, m, H-24), 1.21 and 1.16 (6H, 2s, H-26 and 27), 0.94 (3H, d, H-21, J = 6.0 Hz), 0.89 (9H, s, tBuSi), 0.56 (3H, s, H-18), 0.07 and 0.06 (6H, 2s, MeSi).

### Example 2

### <Synthesis of 3-O-t-butyldimethylsilyl-24-oxo-25-hydroxycholecalciferol>

The (24R)-3-O-t-butyldimethylsilyl-24,25-dihydroxycholecalciferol (637 mg) obtained in Example 1 was dissolved in dimethyl sulfoxide (DMSO) (12.7 ml), and to this solution, triethylamine (1.2 ml) and sulfur trioxide-pyridine (SO₃ · Py) (955 mg) were added. The mixed solution was stirred at 25°C for 30 minutes.

Water was added to the resulting reaction solution under ice cooling and then the solution was extracted thrice with chloroform (40 ml). The recovered organic layer was concentrated under reduced pressure and the residue was purified by silica gel column chromatography (silica gel: 40 g; solvent: n-hexane/ethyl acetate (4/1 v/v)) to obtain the caption compound (514 mg; yield: 81%) as a colorless syrup. The parent peak mass of the mass spectrum of this compound was 528 and the NMR determinations of the compound gave the following results.
¹H-NMR (500 MHz, CDCl₃) δ: 6.16 and 6.02 (2H, 2d, H-6 and 7, J = 11.0 Hz), 5.01 and 4.78 (2H, 2s, H-19), 3.82 (1H, m, H-3), 1.39 (6H, s, H-26 and 27)), 0.94 (3H, d, H-21, J = 6.5 Hz), 0.89 (9H, s, tBuSi), 0.55 (3H, s, H-18), 0.07 and 0.06 (6H, 2s, MeSi).

### Example 3

### <Synthesis of 23,24-dehydro-3-O-t-butyldimethylsilyl-24,25-bis(t-butyldimethylsilyloxy)cholecalciferol>

The 3-O-t-butyldimethylsilyl-24-oxo-25-hydrocholecalciferol (467 mg) obtained in Example 2 was dissolved in tetrahydrofuran (THF) (1.5 ml) and hexamethylphosphoramide (HMPA) (1.5 ml), and to this solution, lithiumdiisopropylamide (LDA) (2.9 ml of a 1.5 M cyclohexane solution) was added at -40°C, followed by stirring for 15 minutes. Then a THF (0.93 ml) solution of TBDMSCl (1.33 g) was added to the resulting solution and stirred at -40°C for one hour.

Water was added to this reaction solution and it was extracted thrice with chloroform (10 ml). The recovered organic layer was concentrated under reduced pressure and the residue was purified by silica gel column chromatography (silica gel: 40 g; solvent: n-hexane/ethyl acetate (20/1 v/v)) to obtain the caption compound (668 mg; yield: 100%) as a colorless syrup. The parent peak mass of the mass spectrum of this compound was 756 and the NMR determinations of the compound gave the following results.
¹H-NMR (500 MHz, CDCl₃) δ: 6.16 and 6.01 (2H, 2d, H-6 and 7, J = 11.0 Hz), 5.00 and 4.78 (2H, 2s, H-19), 4.98 (1H, t, H-23, J = 6.5 Hz), 3.82 (1H, m, H-3), 1.33 (6H, s, H-26, 27), 0.96, 0.91 and 0.89 (27H, 3s, tBuSi), 0.55 (3H, s, H-18), 0.17, 0.16, 0.07 and 0.06 (12H, 4s, MeSi), 0.10 (6H, s, MeSi).

### Example 4

### <Synthesis of 23,25-dihydroxy-24-oxocholecalciferol>

The 23,24-dehydro-3-O-t-butyldimethylsilyl-24,25-bis(t-butyldimethylsilyloxy)cholecalciferol (177 mg) obtained in Example 3 was dissolved in methylene chloride (3.5 ml). To this solution, m-chloroperbenzoic acid (mCPBA) (45 mg) was added at 0°C, and the solution was stirred for one hour. A mixture of a saturated sodium thiosulfate solution and a saturated sodium hydrogencarbonate solution (7:1 v/v) was added to the reaction solution, and the mixed solution was extracted thrice with methylene chloride (5 ml).

The recovered organic layer was concentrated under reduced pressure to obtain a residue containing tris(t-butyldimethylsilyl)ether of 23,25-dihydroxy-24-oxocholecalciferol. This residue was dissolved in THF (3.7 ml), to which tetrabutylammonium fluoride (TBAF) (2.1 ml of a 1.0 M THF solution) was added, followed by stirring of the solution at room temperature for 12 hours. Water was added to the reaction solution and the solution was extracted thrice with ethyl acetate (10 ml). The recovered organic layer was concentrated under reduced pressure.

The residue was purified by silica gel column chromatography (silica gel: 20 g; solvent: n-hexane/ethyl acetate (3/2 v/v)) to obtain the caption compound (62 mg; yield: 62%) (a 2:1 mixture of 23R-form compound and 23S-form compound) as a white solid. The overall yield from 24R,25-dihydroxycholecalciferol was 50% and the parent peak mass of the mass spectrum of the obtained compound was 430 and the NMR determinations of the compound gave the following results.
¹H-NMR (500 MHz, CDCl₃) δ: 6.23 and 6.03 (2H, 2d, H-6 and 7, J = 11.0 Hz), 5.05 and 4.82 (2H, 2s, H-19), 4.70 (1H, m, H-23 (23R)), 4.64 (1H, m, H-23 (23S)), 3.95 (1H, m, H-3), 1.44 and 1.42 (6H, s, H-26 and 27), 1.09 (3H, d, H-21 (23S)), J = 6.5 Hz), 1.06 (3H, d, H-21 (23R), J = 6.5 Hz), 0.59 (3H, s, H-18 (23R)), 0.56 (3H, s, H-18 (23S)).

### Example 5

### <Synthesis of 24-oxo-25-hydroxycholecalciferol>

The 3-O-t-butyldimethylsilyl-24-oxo-25-hydroxycholecalciferol (32.0 mg) obtained in Example 2 was dissolved in THF (0.64 ml). To this solution, tetrabutylammonium fluoride (TBAF) (0.18 ml of a 1.0 M THF solution) was added and the solution was stirred at room temperature for 12 hours. The resulting reaction solution, after addition of water thereto, was extracted thrice with ethyl acetate (3 ml). The recovered organic layer was concentrated under reduced pressure.

The residue was purified by silica gel column chromatography (silica gel: 1.5 g; solvent: n-hexane/ethyl acetate (2/1 v/v)) to obtain the caption compound (23.1 mg; yield: 92%) as a white solid. The overall yield from 24R,25-dihydroxycholecalciferol was 74.5%. The parent peak mass of the mass spectrum of this compound was 414 and the NMR determinations of the compound gave the following results.
¹H-NMR (500 MHz, CDCl₃) δ: 6.23 and 6.04 (2H, 2d, H-6 and 7, J = 11.0 Hz), 5.05 and 4.82 (2H, 2s, H-19), 3.95 (1H, m, H-3),1.39 and 1.38 (6H, s, H-26 and 27), 0.94 (3H, d, H-21, J = 6.5 Hz), 0.55 (3H, s, H-18).

## Claims

1. A process for producing a vitamin D₃ derivative of following formula (I), which comprises oxidizing a compound of following formula (V) to form a compound of following formula (IV), silyl-enol-etherifying the compound of formula (IV) with an enolizing agent and a silylating agent, oxidizing the etherified compound with a peracid, and subjecting the resulting compound to a hydroxyl-protecting group elimination reaction: 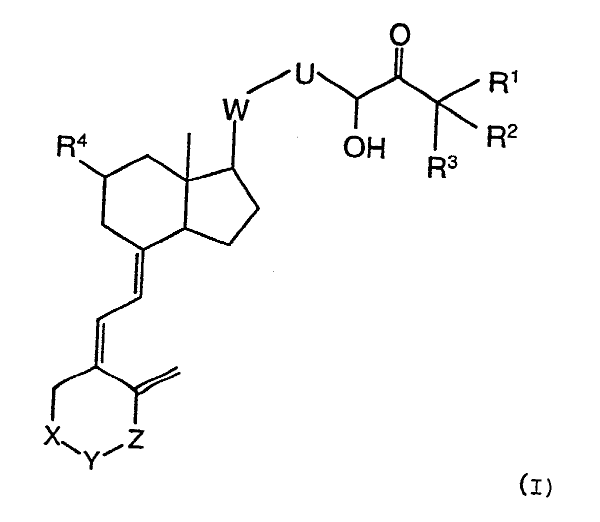 wherein R¹ is an alkyl group, a hydroxyl group, a halogen atom, a hydroxyalkyl group or a halogenated alkyl group; R² and R³ independently are an alkyl group, -OR⁶ (R⁶ being a hydroxyl-protecting group), a halogen atom, a hydroxyalkyl group or a halogenated alkyl group, or R² and R³ together form -O(CH₂)ₙ-, -O(CH₂)ₙO- or -(CH₂)ₙ₊₁- (n being an integer of not less than 1); R⁴ is a hydrogen atom, an alkyl group, a hydroxyl group, a halogen atom, a hydroxyalkyl group, a halogenated alkyl group or an aryl group; U, W, X, Y and Z independently are -(S=O)-, -O-, - (CR⁷R⁸)- or ,(CR⁷R⁸-CR⁹R¹⁰)- (wherein R⁷, R⁸, R⁹ and R¹⁰ independently are a hydrogen atom, an alkyl group, a hydroxyl group, a halogen atom, a hydroxyalkyl group or a halogenated alkyl group; and wherein any hydroxyl group contained in any of U, W, X, Y and Z, is not protected; and wherein in U, W, X, Y and Z, -O- or -(S=O)- does not exist in succession); 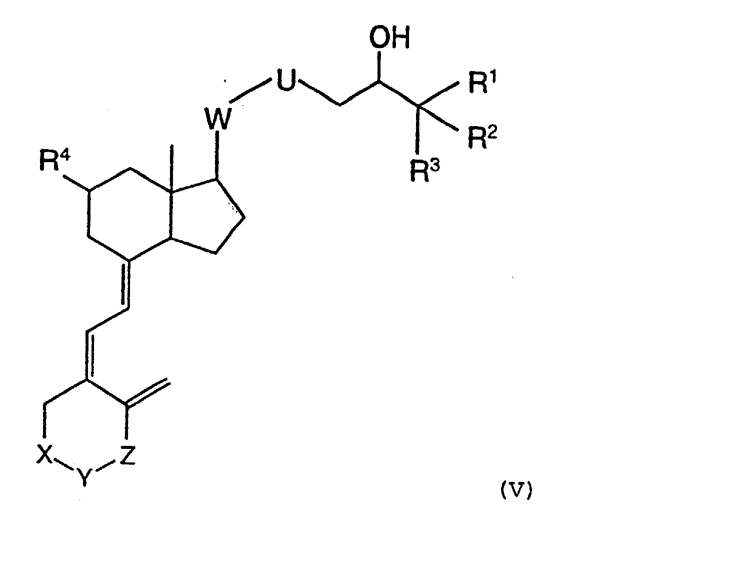 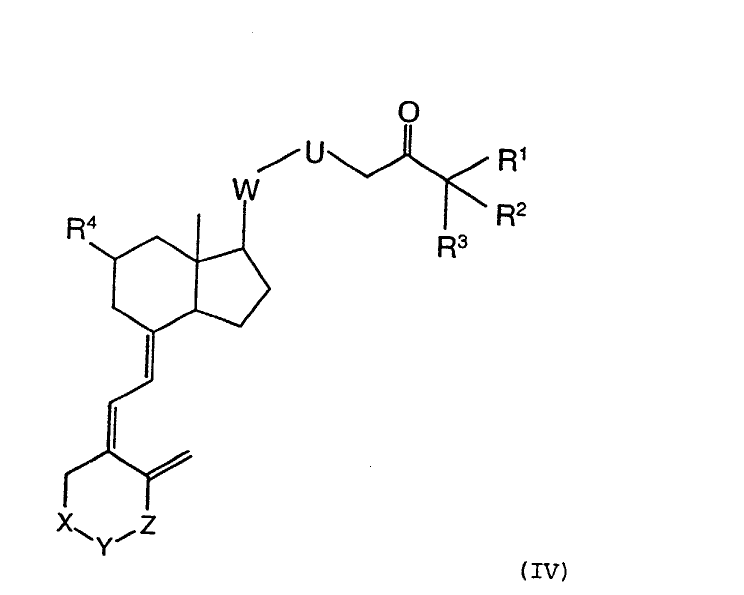 wherein R¹ is an alkyl group, -OR⁵ (R⁵ being a hydrogen atom or a hydroxyl-protecting group), a halogen atom, a hydroxyalkyl group or a halogenated alkyl group; R² and R³ are as defined above; R⁴ is a hydrogen atom, an alkyl group, -OR⁵, a halogen atom, a hydroxyalkyl group, a halogenated alkyl group or an aryl group; U, W, X, Y and Z independently are -(S=O)-, -O-, -(CR⁷R⁸)- or -(CR⁷R⁸-CR⁹R¹⁰) wherein R⁷, R⁸, R⁹ and R¹⁰ independently are a hydrogen atom, an alkyl group, -OR⁵, a halogen atom, a hydroxyalkyl group or a halogenated alkyl group; and wherein any hydroxyl group contained in any of U, W, X, Y and Z is protected; and wherein in U, W, X, Y and Z, -O- or -(S=O)- does not exist in succession).

2. A process according to claim 1, wherein the oxidation of the compound of formula (V) to form the compound of formula (IV) is carried out at 0 to 25°C.

3. A process according to claim 1 or 2 wherein the oxidation of the compound of formula (V) to form the compound of formula (IV) is carried out using an oxidizing agent which is not easily acidified.

4. A process according to any one of the preceding claims wherein the enolizing agent is lithiumdiisopropylamide.

5. A process according to any one of the preceding claims wherein the silylating agent is t-butyldimethylsilyl chloride.

6. A process according to any one of the preceding claims wherein silyl-enol etherification is carried out in tetrahydrofuran, hexaphosphoramide or a mixture thereof.

7. A process according to any one of the preceding claims wherein silyl-enol etherification is carried out at -50° to 25°C.

8. A process according to any one of the preceding claims wherein the peracid is performic acid, peracetic acid, perbenzoic acid, m-chloroperbenzoic acid or perphthalic acid.

9. A process according to any one of the preceding claims wherein the molar ratio of the peracid: silyl-enol ether is 0.8:1 to 1.1:1.

10. A process according to any one of the preceding claims wherein the hydroxyl-protecting group elimination reaction is carried out using tetrabutylammonium fluoride.

11. A process according to any one of the preceding claims wherein the hydroxyl-protecting group elimination reaction is carried out in tetrahydrofuran.

12. A process according to any one of the preceding claims wherein the hydroxyl-protecting group elimination reaction is carried out at 0 to 25°C.

13. A process according to any one of the preceding claims wherein U in formulae (V), (IV) and (I) is -(CR⁷R⁸)-.

14. A process for producing a vitamin D₃ derivative of formula (IV) as defined in claim 1 or 13 which comprises oxidizing a compound of formula (V) as defined in claim 1 or 13.

15. A process according to claim 14 wherein the oxidation is carried out under the conditions of claim 2 or 3.

16. A process for producing a vitamin D₃ derivative of formula (I) as defined in claim 1 or 13 which comprises silyl-enol etherifying a compound of formula (IV) as defined in claim 1 or 13 with an enolizing agent and a silylating agent, oxidizing the etherified compound with a peracid, and subjecting the resulting compound to a hydroxyl-protecting group elimination reaction.

17. A process according to claim 16 which is carried out under the conditions of any one of claims 4 to 12.

18. A process for producing a vitamin D₃ derivative of following formula (Vb) which comprises reacting a compound of following formula (Va) with a compound of following formula (VII): 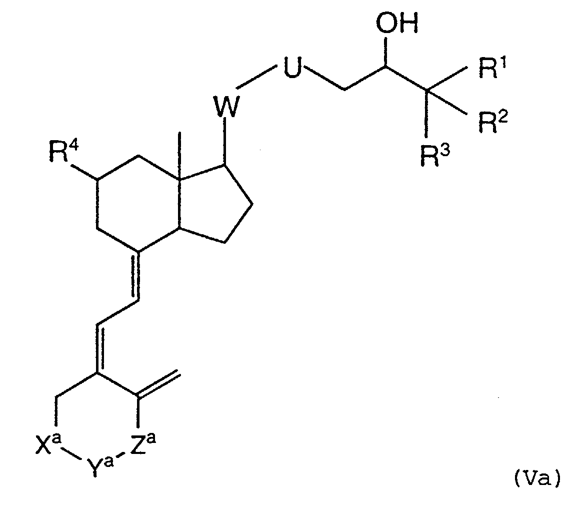
R¹¹R¹²R¹³Si-J (VII) 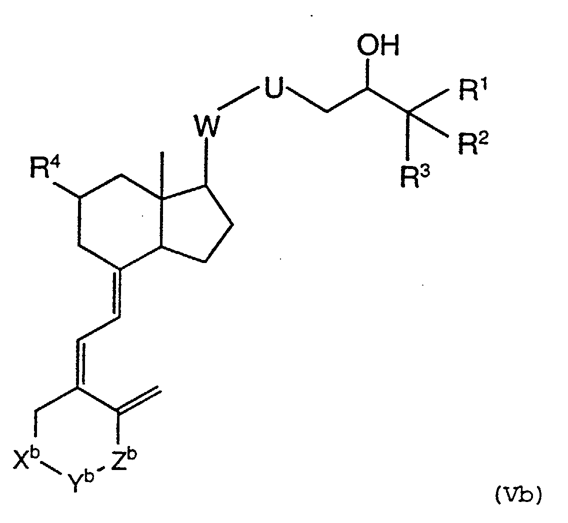
wherein, in formulae (Va) and (Vb), R¹, R², R³ and R⁴ are as defined for formula (IV) in claim 1; U, W, X^{a}, Y^{a}, Z^{a}, X^{b}, Y^{b} and Z^{b} independently are as defined for U, W, X, Y and Z in formula (IV) in claim 1, at least one of X^{a}, Y^{a}, and Z^{a} being -CH(OH)- and at least one of X^{b}, Y^{b} and Z^{b} being -CH(OSiR¹³R¹²R¹¹)- (wherein R¹¹, R¹² and R¹³ independently are an alkyl group having 1 to 6 carbon atoms); J is a halogen atom and -SiR¹³R¹²R¹¹ represents a hydroxyl-protecting group; and wherein in U, W, X^{a}, Y^{a} and Z^{a} or in U, W, X^{b}, Y^{b} and Z^{b}, -O- or -(S=O)- does not exist in succession.

19. A process according to claim 18 wherein the compound of formula (VII) is t-butyldimethylsilyl chloride.

20. A process according to claim 18 or 19 wherein U in formulae (Va) and (Vb) is -(CR⁷R⁸)-.
